(19) Europäisches Patentamt
European Patent Office
Office européen des brevets

(11) Numéro de publication: 0 183 577
B1

# (12) FASCICULE DE BREVET EUROPEEN

(45) Date de publication du fascicule du brevet: 07.02.90

(51) Int. Cl.⁴: C07D 285/12, A61K 31/41

(21) Numéro de dépôt: 85401972.6

(22) Date de dépôt: 10.10.85

(54) **Dérivés du thiadiazole actifs sur le système nerveux central, procédé d'obtension et compositions pharmaceutiques les contenant.**

(30) Priorité: 18.10.84 FR 8415991

(43) Date de publication de la demande: 04.06.86 Bulletin 86/23

(45) Mention de la délivrance du brevet: 07.02.90 Bulletin 90/6

(84) Etats contractants désignés: AT BE CH DE FR GB IT LI LU NL SE

(56) Documents cités:
FR-A-2 092 714
US-A-3 919 428

JOURNAL OF PHARMACEUTICAL SCIENCES, vol. 64, no. 7, juillet 1975, pages 1250-1252, Washington, US; I. LALEZARI et al.: "Synthesis and pharmacological activity of 5-substituted 2-(N,N-dialkylaminoethyl)amino- and 2-N-methylpiperazinyl-1,3,4-thiadiazoles" 000

(73) Titulaire: SANOFI, 40, Avenue George V, F-75008 Paris(FR)

(72) Inventeur: Brodin, Roger, 10, Rue de la Goelette Mas de Neuville, F-34100 Montpellier(FR)
Inventeur: Wermuth, Camille Georges, 3, Rue de la Côte d'Azur, F-67100 Strasbourg(FR)
Inventeur: Worms, Paul, 10, Rue du Vevois, F-34980 Saint Gely du Fesc(FR)

(74) Mandataire: Gillard, Marie-Louise et al, Cabinet Beau de Loménie 55, Rue d'Amsterdam, F-75008 Paris(FR)

EP 0 183 577 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne en tant que produits nouveaux des dérivés du thiadiazole présentant d'intéressantes propriétés sur le système nerveux central. Elle concerne également un procédé d'obtention desdits dérivés et les compositions pharmaceutiques les contenant.

Plus précisément, les composés selon l'invention répondent à la formule générale I:

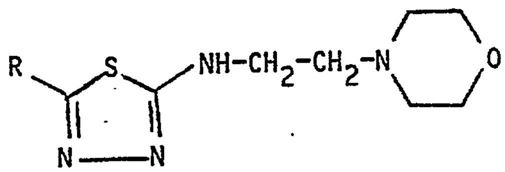

(I)

dans laquelle R représente :
- un groupe alkyle droit ou ramifié ayant de 1 à 5 atomes de carbone;
- un groupe cycloalkyle ayant 5 ou 6 atomes de carbone;
- un groupe phényle éventuellement substitué par 1 ou 2 atomes d'halogène, de préférence le chlore, par 1 ou 2 groupes alkyle ou alcoxy ayant de 1 à 5 atomes de carbone ou par 1 ou 2 groupes hydroxy ou par un groupe nitro ou trifluorométhyle;
- un groupe biphénylyle;
- un groupe α-naphtyle.

Les sels que donnent les composés de formule générale I avec les acides minéraux ou organiques pharmaceutiquement acceptables font partie intégrante de l'invention.

Des composés voisins des composés selon l'invention ont été décrits dans la littérature chimique. Ainsi, dans Journal of Pharmaceutical Sciences 64 (7), 1250-1252, (1975), il est mentionné des composés répondant à la formule générale II:

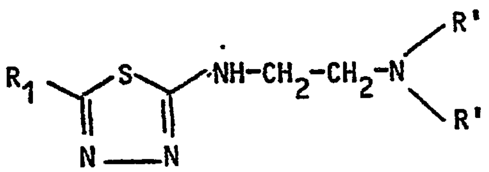

(II)

dans laquelle R' représente un groupe méthyle ou éthyle et $R_1$ représente un groupe tolyle, méthoxyphényle ou nitrophényle.

Ces composés sont décrits comme présentant des propriétés antihistaminiques et anticholinergiques.

D'une façon surprenante, il a été trouvé que les composés de l'art antérieur ne possèdent que très faiblement les propriétés sur le système nerveux central dont sont dotés les composés selon l'invention.

Les composés selon l'invention peuvent être préparés à partir des halogéno-2 thiadiazoles-1,3,4 convenablement substitués en position 5 selon le schéma réactionnel :

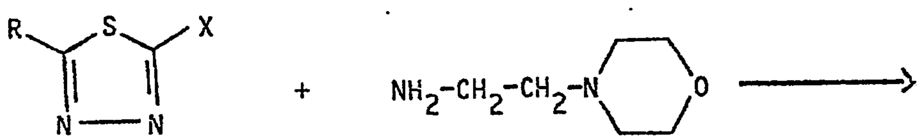

\+ (I)

1 2

(X = Cl ou Br)

La réaction est effectuée par chauffage des deux réactifs au sein d'un solvant tel qu'un alcanol comme l'éthanol ou le n-butanol. Le plus souvent, on opère avec un excès de l'amine 2 pour fixer l'hydracide formé au cours de la réaction.

Enfin, lorsque X est le chlore, on peut faciliter la réaction en opérant en présence d'iodure de potassium.

Le composé (I) est isolé par extraction en milieu acide dilué et la base est libérée par alcalinisation de la solution aqueuse. Eventuellement, le produit ainsi isolé peut être salifié par les méthodes habituelles.

Lorsque R représente un groupe hydroxyphényle ou dihydroxyphényle, les composés (I) s'obtiennent par déméthylation des composés (I) correspondants où R représente un groupe méthoxyphényle ou diméthoxyphényle selon un procédé connu, par exemple par chauffage avec l'acide bromhydrique concentré.

Les produits de départ halogénés 1 sont connus ou peuvent être préparés par des méthodes connues. Ainsi, les produits 1 peuvent être obtenus à partir des composés aminés correspondants :

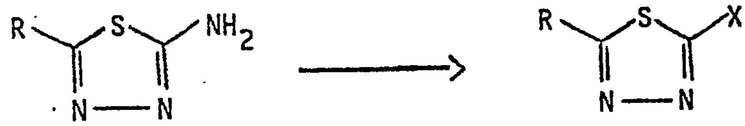

3 1

par diazotation et décomposition du sel de diazonium en présence de l'hydracide XH selon les méthodes décrites dans Chemische Berichte 89, 1534-1543, (1956) et Tetrahedron 24, 3209-3217, (1968).

Les composés aminés 3 sont connus ou peuvent être préparés selon des procédés connus [Journal of Pharmaceutical Society of Japan 72, 373-375, (1952); Journal of the Chemical Society (1949), 1163-1167 et Canadian Journal of Chemistry 37, 1121-1123, (1959)] qui consistent à transformer l'acide RCOOH ou le chlorure d'acide RCOCl en thiosemicarbazide correspondant et à cycliser ce dernier par un agent de déshydratation tel que l'acide sulfurique.

Les exemples suivants non limitatifs permettront de mieux comprendre l'invention.

EXEMPLE 1

(Morpholino-2 éthylamino)-2 phényl-5 thiadiazole-1,3,4, dichlorhydrate. (I R = $C_6H_5$); SR 95311 A.

Le chloro-2 phényl-5 thiadiazole-1,3,4 utilisé comme produit de départ est préparé comme indiqué dans Tetrahedron 24, 3214, (1968).

A la solution de 3,93 g du chloro-2 phényl-5 thiadiazole- 1,3,4 dans 30 ml de butanol-1, on ajoute 5,2 g de morpholino-2 éthylamine et 0,01 g d'iodure de potassium.

On chauffe sous agitation au reflux pendant 2 heures puis on élimine le solvant sous vide. On reprend le résidu dans l'acétate d'éthyle et épuise la solution organique avec une solution aqueuse d'acide chlorhydrique à 10%. On alcalinise la phase aqueuse jusqu'à pH 8-9 par addition de bicarbonate de sodium. On extrait avec de l'acétate d'éthyle, lave la solution organique deux fois avec de l'eau puis sèche sur sulfate de magnésium. On évapore le solvant sous vide. Le résidu huileux est dissous à chaud dans le minimum (3 à 4 ml) d'isopropanol puis on ajoute à la solution 2 ml de solution concentrée d'acide chlorhydrique. Il se sépare un précipité incolore qu'on essore, sèche sous vide et recristallise dans le méthanol.

Finalement, on obtient un solide incolore. Poids : 2,9 g; F. : 222-224°C.

On obtient le même produit en remplaçant le dérivé chloré de départ par une quantité équivalente du dérivé bromé correspondant. Dans ce cas, il est inutile d'ajouter de l'iodure de potassium.

EXEMPLE 2

(Morpholino-2 éthylamino)-2 cyclohexyl-5 thiadiazole-1,3,4. (I R = $C_6H_{11}$); SR 43058.

a) Amino-2 cyclohexyl-5 thiadiazole-1,3,4

En refroidissant au bain d'eau, on mélange 25,6 g d'acide cyclohexane carboxylique, 21 ml d'acide sulfurique concentré et 15,2 g de thiosemicarbazide. On chauffe ensuite le mélange sous agitation à 90-100°C pendant 15 heures.

Après refroidissement, on verse le mélange dans 400 ml d'eau froide et sépare un insoluble. La solution aqueuse est alcalinisée par 150 ml d'ammoniaque concentrée. On essore le précipité, lave avec de l'eau, sèche et recristallise dans l'éthanol.

F. : 254-256°C, Poids : 14 g.

b) Bromo-2 cyclohexyl-5 thiadiazole-1,3,4

A une suspension refroidie à -20°C de 2,6 g de poudre de cuivre dans 200 ml de solution d'acide bromhydrique à 48%, on ajoute par petites fractions, en environ 1 heure, le mélange intime finement broyé de 60 g de nitrite de sodium et 13,8 g d'amino-2 cyclohexyl-5 thiadiazole-1,3,4.

Pendant toute la durée de l'addition, la température est maintenue entre -20 et -10°C. L'addition terminée, on retire le bain réfrigérant et agite à température ambiante pendant 2 heures puis à 40-45°C pendant 1 heure 30 minutes. On laisse une nuit au repos puis on ajoute 500 ml d'eau et extrait avec de l'acétate d'éthyle. On lave la phase organique avec de l'eau salée puis on neutralise par addition de soude concentrée. On décante la phase organique, lave avec de l'eau salée et sèche sur sulfate de magnésium. On évapore le solvant à siccité. Il reste une huile (13 g) qui concrétise lentement et sera utilisée telle quelle pour l'étape suivante.

c) SR 43058

A partir du dérivé bromé obtenu ci-dessus, on opère comme dans l'exemple 1.
Dans ce cas, la base obtenue par alcalinisation au bicarbonate de sodium cristallise après évaporation du solvant.
On ne procède donc pas à la salification mais recristallise dans l'éther isopropylique. F. : 102-104°C.

EXEMPLES 3 à 16

En opérant comme dans les exemples précédents, mais en faisant varier le produit de départ, on obtient les produits (I) figurant dans le tableau suivant.

TABLEAU I

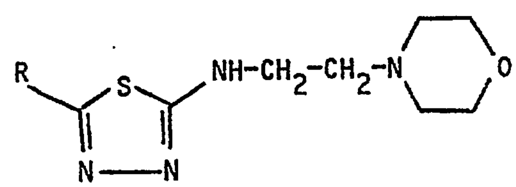

| N° Code SR | R | Base ou Sel | Point de fusion °C (Solvant de cristallisation) |
|---|---|---|---|
| 43055 A | $- CH_3$ | Chlorhydrate | 246-248 (éthanol) |
| 43056 A | 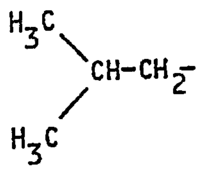 | Dimaléate | 105-107 (isopropanol) |
| 43057 A | 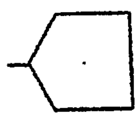 | Maléate | 156-158 (éthanol) |
| 95340 A | 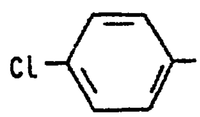 | Dichlorhydrate | 235-237 (méthanol) |
| 95396 A | 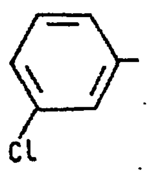 | Dichlorhydrate | 240-242 (méthanol) |
| 95397 A | 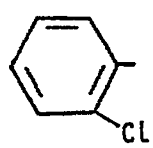 | Dichlorhydrate | 235-240 (méthanol) |
| 95371 A | 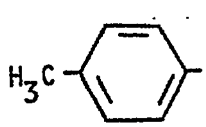 | Dichlorhydrate | 217-219 (méthanol) |

| | | | |
|---|---|---|---|
| 43531 A | 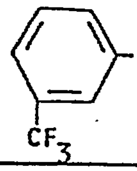 | Dichlorhydrate | 210 (éthanol-méthanol) |
| 43604 | 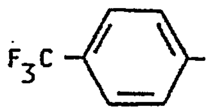 | Base | 135 (méthanol) |
| 43530 | 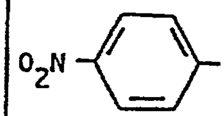 | Base | 224 (éthanol) |
| 43198 | 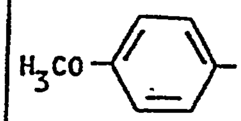 | Base | 110-112 (éthanol) |
| 43295 | 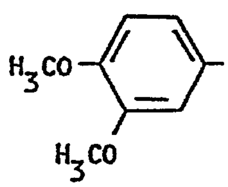 | Base | 122-124 (acétate d'éthyle) (avec 1 $H_2O$) |
| 43294 | 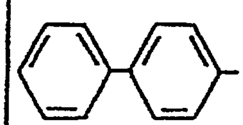 | Base | 160-162 (éthanol) (avec 0,5 $H_2O$) |
| 95559 A | 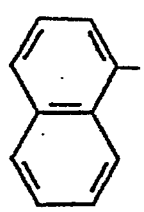 | Dichlorhydrate | 175-177 (méthanol) |

EXEMPLE 17

(Morpholino-2 éthylamino)-2 (hydroxy-4 phényl)-5 thiadiazole-1,3,4, dichlorhydrate.

(I) R = 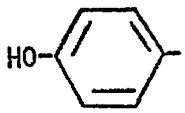 ; SR 43202 A

On chauffe au reflux sous agitation pendant 20 heures le mélange de 7,1 g de (morpholino-2 éthylamino)-2 (méthoxy-4 phényl)-5 thiadiazole-1,3,4 (SR 43198, exemple 13) et 150 ml de solution d'acide bromhydrique à 48%.

On ajoute 200 ml de solution de soude à 30%. On lave la phase aqueuse avec 500 ml d'acétate d'éthyle puis on y ajoute 500 ml de solution saturée de chlorure d'ammonium et laisse 2 heure sous agitation. On essore le précipité et lave avec de l'eau. On dissout le solide dans 100 ml d'éthanol et ajoute 9,2 ml d'acide chlorhydrique concentré. On essore le chlorhydrate et recristallise dans le méthanol.

On obtient 5 g du dichlorhydrate qui cristallise avec 0,5 molécule d'eau; F. : 250-252°C.

EXEMPLE 18

(Morpholino-2 éthylamino)-2 (dihydroxy-3,4 phényl)-5 thiadiazole-1,3,4, dichlorhydrate.

(I) R = 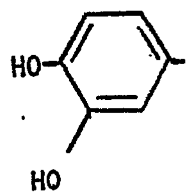 ; SR 43296 A

On opère comme dans l'exemple 17 à partir de (morpholino-2 éthylamino)-2 (diméthoxy-3,4 phényl)-5 thiadiazole- 1,3,4 (SR 43295, exemple 14).

De la même façon, on obtient SR 43296 A; F. : 161-163°C; qui cristallise avec 1 molécule d'eau.

Les composés selon l'invention ont été étudiés en ce qui concerne leurs propriétés thérapeutiques et plus spécialement leur activité sur le système nerveux central.

Dans cette étude, les produits selon l'invention ont été comparés à deux composés voisins substitués en position 2 par une chaîne dialkylamino éthylamino identique à celle des composés connus dans l'art antérieur.

Ces composés répondent aux formules :

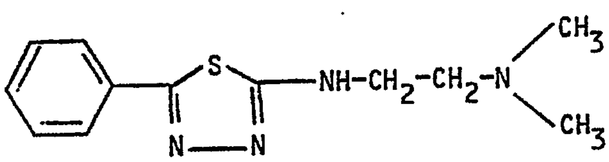 , 2 HCl

Composé A

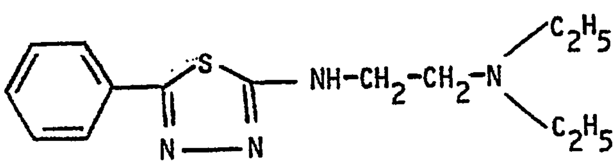 , 2 HCl

Composé B

TOXICITE AIGUE

Les produits à étudier ont été administrés par voie intrapéritonéale à doses croissantes à des lots de 10 souris (CD1, Charles River; 20 ± 1 g). La mortalité provoquée par les produits étudiés a été notée pendant les 24 heures ayant suivi l'administration du produit.

A partir des résultats obtenus, on a déterminé pour chacun des produits étudiés la dose létale 50, c'est-à-dire la dose provoquant la mort de 50% des animaux.

Tous les produits selon l'invention possèdent une dose létale 50 supérieure ou égale à 300 mg/kg, alors que,pour les composés de comparaison, la mortalité à la dose de 300 mg/kg est de 70% pour le composé A et de 90% pour le composé B.

ANTAGONISME DE LA PTOSE INDUITE PAR LA RESERPINE

Ce test décrit par GOURET et Coll. (Journal de Pharmacologie, Paris, 8, 333-350, 1977) a été réalisé chez la souris femelle CD1 (Charles River) pesant 20 ± 1 g. La réserpine entraîne un ptôsis 1 heure après son administration intraveineuse; la plupart des antidépresseurs s'opposent à ce ptôsis.

Le protocole suivant a été choisi. Les substances à étudier ont été administrées i.p. et la réserpine a été administrée simultanément par voie intraveineuse à la dose de 2 mg/kg. 1 heure après l'administration de réserpine, on a noté le nombre d'animaux ne présentant pas de ptôsis.

Ce test a été réalisé sur des lots de 10 souris; les résultats sont exprimés en $DE_{50}$ (dose efficace 50%), soit la dose qui bloque complètement le ptôsis réserpinique chez 50% d'animaux.

Avec divers produits selon l'invention, les résultats obtenus ont été les suivants :

SR 95311 A $DE_{50}$ : 4,6 mg/kg
SR 95340 A $DE_{50}$ : 8,8 mg/kg
SR 95396 A $DE_{50}$ : 11,3 mg/kg
SR 95397 A $DE_{50}$ : 18 mg/kg
SR 43058 $DE_{50}$ : 22 mg/kg
SR 43057 A $DE_{50}$ : 23 mg/kg

Dans les mêmes conditions, les produits de comparaison ont donné les résultats suivants :

Composé A $DE_{50}$ : 32 mg/kg
Composé B $DE_{50}$ : 43 mg/kg

POTENTIALISATION DES TREMBLEMENTS INDUITS PAR LE L-5-HTP

Le L-5-HTP (L-5-hydroxytryptophane) est le précurseur de biosynthèse de la sérotonine. Son injection chez la souris provoque un syndrome comportemental caractérisé en particulier par un fort tremblement. Les antidépresseurs inhibiteurs de la recapture de sérotonine, ou inhibiteurs de la monoamine oxydase (IMAO),potentialisent cet effet du L-5-HTP (LESSIN, 1959).

Le protocole décrit par LESSIN (Biochem. Pharmacol., 2, 290-298, 1959) a été utilisé. Les produits à étudier ont été administrés par voie i.p. à des souris femelles CD1 (Charles River; 20 ± 1 g) 1 heure avant une injection de L-5-HTP à la dose de 200 mg/kg i.p. (dose maximale n'induisant pas de tremblement). L'absence ou la présence d'un tremblement net a été observée pendant les 20 minutes suivantes.

Les résultats sont exprimés en $DE_{50}$, c'est-à-dire la dose induisant les tremblements chez 50% des animaux traités.

Les résultats suivants ont été obtenus :

SR 95311 A $DE_{50}$ : 6 mg/kg
SR 95340 A $DE_{50}$ : 3,6 mg/kg
SR 43058 $DE_{50}$ : 17 mg/kg
SR 43056 A $DE_{50}$ : 12 mg/kg
SR 95397 A $DE_{50}$ : 14,5 mg/kg
SR 43531 A $DE_{50}$ : 7,8 mg/kg
SR 43530 $DE_{50}$ : 6,2 mg/kg
SR 43198 $DE_{50}$ : 7 mg/kg

alors que, avec les deux produits de comparaison, la $DE_{50}$ est supérieure à 60 mg/kg.

COMPORTEMENT ROTATOIRE

Ce test est celui décrit par PROTAIS et Coll. (Journal de Pharmacologie, Paris, 7, 251-255, 1976). Des souris femelles (Charles River, CD1) pesant de 25 à 30 g ont fait préalablement l'objet d'une lésion unilatérale du striatum par injection stéréotaxique de 6-hydroxydopamine à raison de 8 µg par animal. Une semaine après cette opération, les produits ont été administrés par voie i.p. à des groupes de 7 souris. Le nombre de rotations a été évalué pendant 2 minutes 1 heure aprés l'administration du produit. Les rotations ipsilatérales à la lésion ont été comptées positivement, celles contralatérales comptées négativement.

La somme algébrique des rotations pour un groupe d'animaux traités a été comparée à celle du groupe d'animaux témoins n'ayant reçu que le véhicule (eau gommée).

Dans ce test, les stimulants des récepteurs dopaminergiques (type apomorphine) diminuent les rotations spontanées, alors que les agents dopaminomimétiques indirects (type amphétamine) les augmentent.

Les résultats sont exprimés en D.E.M. (dose efficace minimale) ou dose minimale nécessaire pour diminuer significativement (test du t Student) le nombre de rotations ipsilatérales spontanément observées chez les témoins.

Les résultats suivants ont été obtenus :

SR 95311 A D.E.M. : 0,1 mg/kg
SR 95396 A D.E.M. : 0,1 mg/kg
SR 95397 A D.E.M. : 0,1 mg/kg
SR 43604 D.E.M. : 0,1 mg/kg
SR 43530 D.E.M. : 0,1 mg/kg
SR 43202 A D.E.M. : 0,1 mg/kg
SR 43295 D.E.M. : 0,1 mg/kg
SR 43296 A D.E.M. : 0,1 mg/kg
SR 43294 D.E.M. : 0,1 mg/kg
SR 95559 A D.E.M. : 0,1 mg/kg

alors que, pour les deux produits de comparaison, la D.E.M. est supérieure à 2 mg/kg.

Suivant ces résultats, les produits selon l'invention présentent des propriétés antidépressives et dopaminomimétiques, alliées à un coefficient thérapeutique très favorable.

Par suite, les produits selon l'invention peuvent être utilisés en thérapeutique humaine dans diverses affections neurologiques et psychiatriques : traitement des troubles de l'humeur et du comportement, dépressions névrotiques et endogènes, troubles de la mémoire chez les sujets âgés, hyperkinésie infantile, autisme, insuffisance sexuelle d'origine psychogène, maladie de Parkinson.

Ces produits peuvent être administrés par voie orale ou par voie parentérale.

Ainsi, la présente invention a également pour objet les compositions pharmaceutiques contenant, à titre d'ingrédients actifs, un composé selon l'invention en combinaison avec un véhicule pharmaceutiquement acceptable.

La posologie peut varier dans de larges proportions, en particulier suivant le type et la gravité de l'affection à traiter et suivant la voie d'administration. En général, chez l'adulte, la posologie par voie orale variera entre 1 et 500 mg par jour, en une ou plusieurs prises.

A titre d'exemple de préparation galénique, on peut indiquer des gélules contenant :

| | |
|---|---|
| **SR 95311 A** | **50 mg** |
| **Aerosil** | **0,5 mg** |
| **Stéarate de magnésium** | **1,5 mg** |
| **Amidon STA RX 1500** | **48 mg** |
| | **100 mg** |

**Revendications pour les Etats contractants BE, CH, DE, FR, GB, IT, LI, LU, NL, SE**

1. Dérivés du thiadiazole répondant à la formule générale I:

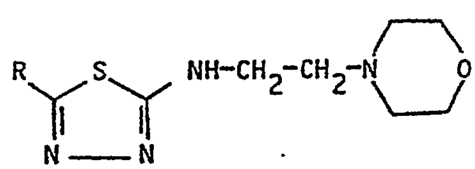

(I)

dans laquelle R représente:
- un groupe alkyle droit ou ramifié ayant de 1 à 5 atomes de carbone;
- un groupe cycloalkyle ayant 5 ou 6 atomes de carbone;
- un groupe phényle, éventuellement substitué par 1 ou 2 atomes d'halogène de préférence le chlore, par 1 ou 2 groupes alkyle ou alcoxy ayant de 1 à 5 atomes de carbone ou par 1 ou 2 groupes hydroxy ou par un groupe nitro ou trifluorométhyle;
- un groupe biphénylyle;
- un groupe alpha-naphtyle;

et les sels pharmaceutiquement acceptables desdits dérivés.

2. Procédé pour l'obtention des dérivés du thiadiazole selon la revendication 1, caractérisé en ce qu'il consiste à faire réagir un halogéno-2 thiadiazole-1,3,4 de formule

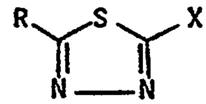

dans laquelle X représente le chlore ou le brome et R est tel que défini à la revendication 1, mais différent d'un groupe hydroxyphényle ou dihydroxyphényle, avec une amine de formule:

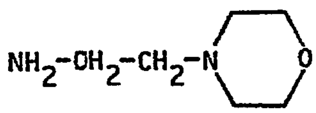 ;

à extraire le dérivé résultant du milieu réactionnel; à éventuellement déméthyler, selon un procédé connu, les dérivés obtenus de formule générale I dans laquelle R représente un groupe méthoxyphényle ou diméthoxyphényle pour obtenir ceux de formule générale I dans laquelle R représente un groupe hydroxyphényle ou dihydroxyphényle; et éventuellement à transformer le composé obtenu en l'un de ses sels pharmaceutiquement acceptable.

3. Procédé selon la revendication 2, caractérisé en ce que la réaction entre l'halogéno-2 thiadiazole-1,3,4 et l'amine est effectuée par chauffage des réactifs au sein d'un solvant.

4. Procédé selon l'une des revendications 2 ou 3, caractérisé en ce que la réaction entre l'halogéno-2 thiadiazole-1,3,4 et l'amine est effectuée avec un excès de l'amine.

5. Composition pharmaceutique, caractérisée en ce qu'elle contient à titre d'ingrédient actif un dérivé du thiadiazole selon la revendication 1, en combinaison avec un véhicule pharmaceutiquement acceptable.

6. Composition pharmaceutique, active sur le système nerveux central, caractérisée en ce qu'elle contient à titre d'ingrédient actif un dérivé du thiadiazole selon la revendication 1, en combinaison avec un véhicule pharmaceutiquement acceptable.

**Revendications pour l'état contractant: AT**

1. Procédé pour la préparation de dérivés du thiadiazole répondant à la formule générale I:

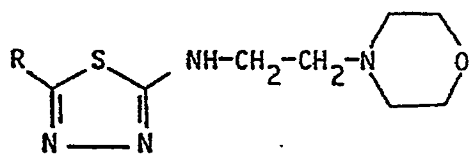 (I)

dans laquelle R représente:
- un groupe alkyle droit ou ramifié ayant de 1 à 5 atomes de carbone;
- un groupe cycloalkyle ayant 5 ou 6 atomes de carbone;
- un groupe phényle, éventuellement substitué par 1 ou 2 atomes d'halogène de préférence le chlore, par 1 ou 2 groupes alkyle ou alcoxy ayant de 1 à 5 atomes de carbone ou par 1 ou 2 groupes hydroxy ou par un groupe nitro ou trifluorométhyle;
- un groupe biphénylyle;
- un groupe alpha-naphtyle;

et les sels pharmaceutiquement acceptables desdits dérivés,
caractérisé en ce qu'il consiste à faire réagir un halogéno-2 thiadiazole-1,3,4 de formule

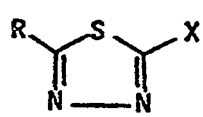

dans laquelle X représente le chlore ou le brome et R est tel que défini ci-dessus, mais différent d'un groupe hydroxyphényle ou dihydroxyphényle, avec une amine de formule:

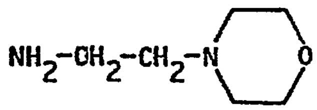 ;

à extraire le dérivé résultant du milieu réactionnel; à éventuellement déméthyler, selon un procédé connu, les dérivés obtenus de formule générale I dans laquelle R représente un groupe méthoxyphényle ou diméthoxyphényle pour obtenir ceux de formule générale I dans laquelle R représente un groupe hydroxyphényle ou dihydroxyphényle; et éventuellement à transformer le composé obtenu en l'un de ses sels pharmaceutiquement acceptable.

2. Procédé selon la revendication 2, caractérisé en ce que la réaction entre l'halogéno-2 thiadiazole-1,3,4 et l'amine est effectuée par chauffage des réactifs au sein d'un solvant.

3. Procédé selon l'une des revendications 1 ou 2, caractérisé en ce que la réaction entre l'halogéno-2 thiadiazole-1,3,4 et l'amine est effectuée avec un excès de l'amine.

4. Utilisation des dérivés du thiadiazole obtenus par un procédé selon l'une quelconque des revendications 1 à 3, en combinaison avec un véhicule pharmaceutiquement acceptable, pour la préparation de compositions pharmaceutiques.

5. Utilisation des dérivés du thiadiazole obtenus par un procédé selon l'une quelconque des revendications 1 à 3, en combinaison avec un véhicule pharmaceutiquement acceptable, pour la préparation de compositions pharmaceutiques, active sur le système nerveux central.

**Claims for contracting States: BE, CH, DE, FR, GB, IT, LI, LU, NL, SE**

1. Thiadiazole derivatives of general formula I

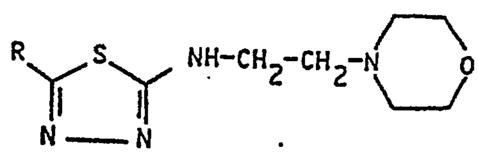 (I)

in which represents:

- a linear or branched alkyl group having 1 to 5 carbon atoms;
- a cycloalkyl group having 5 to 6 carbon atoms;
- a phenyl group optionally substituted by 1 or 2 halogen atoms, preferably chlorine, by 1 or 2 alkyl or alkoxy groups having 1 to 5 carbon atoms, by 1 or 2 hydroxyl groups or by a nitro or trifluoromethyl group;
- a biphenylyl group; or
- an alpha-naphthyl group;

and the pharmaceutically acceptable salts of said derivatives.

2. Process for the preparation of the thiadiazole derivatives according to claim 1, characterized in that it consists in reacting a 2-halogeno-1,3,4-thiadiazole of the formula:

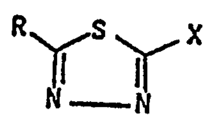

in which X represents chlorine or bromine and R is as defined in claim 1, but is other than a hydroxyphenyl or dihydroxyphenyl group, with an amine of the formula:

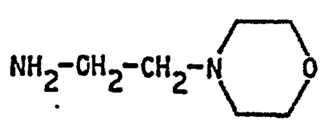

in extracting the resulting derivative from the reaction medium; if appropriate demethylating, by a known process, the resulting derivatives of general formula (I) in which R represents a methoxyphenyl or dimethoxyphenyl group, to give the derivatives of general formula (I) in which R represents a hydroxyphenyl or dihydroxyphenyl group, and if appropriate converting the resulting compound to one of its pharmaceutically acceptable salts.

3. Process according to claim 2, characterized in that the reaction of the 2-halogeno-1,3,4-thiadiazole with the amine is carried out by heating the reactants in a solvent.

4. Process according to one of claims 2 or 3, characterized in that the reaction of the 2-halogeno-1,3,4-thiadiazole with the amine is carried out with an excess of the amine.

5. Pharmaceutical composition, characterized in that it contains a thiadiazole derivative according to claim 1 as the active ingredient, in combination with a pharmaceutically acceptable vehicle.

6. Pharmaceutical composition, active on the central nervous system, characterized in that it contains as active ingredient a thiadiazole derivative according to claim 1, in combination with a pharmaceutically acceptable vehicle.

**Claims for the Contracting State AT**

1. Process for the preparation of thiadiazole derivatives of general formula I:

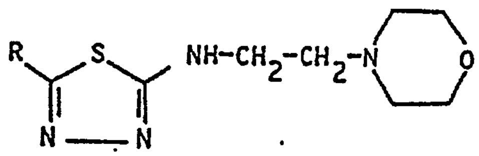

(I)

in which R represents:

– a linear or branched alkyl group having 1 to 5 carbon atoms;

– a cycloalkyl group having 5 to 6 carbon atoms;

– a phenyl group optionally substituted by 1 or 2 halogen atoms, preferably chlorine, by 1 or 2 alkyl or alkoxy groups, having 1 to 5 carbon atoms, by 1 or 2 hydroxyl groups or by a nitro or trifluoromethyl group;

– a biphenylyl group; or

– an alpha-naphthyl group;

and of the pharmaceutically acceptable salts of said derivatives, characterized in that it consists in reacting a 2-halogeno-1,3,4-thiadiazole of the formula:

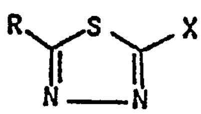

in which X represents chlorine or bromine and R is as defined above but is other than a hydroxyphenyl or dihydroxyphenyl group, with an amine of the formula:

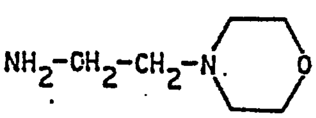

in extracting the resulting derivative from the reaction medium, if appropriate demethylating, by a known process, the resulting derivatives of general formula (I) in which R represents a methoxyphenyl or dimethoxyphenyl group, to give the derivatives of general formula (I) in which R represents a hydroxyphenyl or dihydroxyphenyl group, and if appropriate converting the resulting compound to one of its pharmaceutically acceptable salts.

2. Process according to claim 1, characterized in that the reaction of the 2-halogeno-1,3,4-thiadiazole with the amine is carried out by heating the reactants in a solvent.

3. Process according to claims 1 or 2, characterized in that the reaction of the 2-halogeno-1,3,4-thiadiazole with the amine is carried out with an excess of the amine.

4. Use of the thiadiazole derivatives obtained with a process according to any one of claims 1 to 3, in combination with a pharmaceutically acceptable vehicle for the preparation of pharmaceutical compositions.

5. Use of the thiadiazole derivatives obtained with a process according to any one of claims 1 to 3, in combination with a pharmaceutically acceptable vehicle for the preparation of pharmaceutical compositions, active on the central nervous system.

**Patentansprüche für die Vertragsstaaten BE, CH, DE, FR, GB, IT, LI, LU, NL, SE:**

1. Thiadiazolderivate der allgemeinen Formel I:

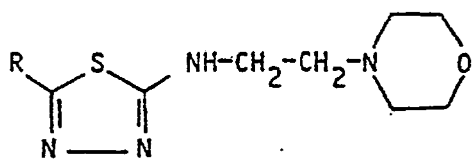

(I)

worin R darstellt:
- eine gerade oder verzweigte Alkylgruppe mit 1 bis 5 Kohlenstoffatomen;
- eine Cycloalkylgruppe mit 5 oder 6 Kohlenstoffatomen;
- eine Phenylgruppe, gegebenenfalls substituiert durch 1 oder 2 Halogenatome, vorzugsweise Chlor, durch 1 oder 2 Alkyl- oder Alkoxygruppen mit 1 bis 5 Kohlenstoffatomen oder durch 1 oder 2 Hydroxygruppen oder durch eine Nitro- oder Trifluormethylgruppe;
- eine Biphenylylgruppe;
- eine alpha-Naphthylgruppe;

und die pharmazeutisch akzeptablen Salze dieser Derivate.

2. Verfahren zur Herstellung der Thiadiazolderivate nach Anspruch 1, dadurch gekennzeichnet, daß es darin besteht, daß ein 2-Halogen-1,3,4-thiadiazol der Formel

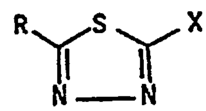

worin X Chlor oder Brom darstellt und R wie in Anspruch 1 definiert, aber keine Hydroxyphenyl- oder Dihydroxyphenylgruppe ist, mit einem Amin der Formel

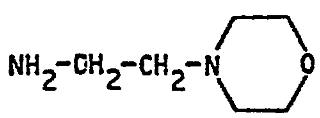

zur Umsetzung gebracht wird, das resultierende Derivat aus dem Reaktionsmilieu extrahiert wird, die erhaltenen Derivate der allgemeinen Formel I, worin R eine Methoxyphenyl- oder Dimethoxyphenylgruppe darstellt, gegebenenfalls nach einem bekannten Verfahren entmethyliert werden, um jene der allgemeinen Formel I zu erhalten, in welcher R für eine Hydroxyphenyl- oder Dihydroxyphenylgruppe steht, und gegebenenfalls die erhaltene Verbindung in eines ihrer pharmazeutisch akzeptablen Salze übergeführt wird.

3. Verfahren nach Anspruch 2, dadurch gekennzeichnet, daß die Reaktion zwischen dem 2-Halogen-1,3,4-thiadiazol und dem Amin durch Erhitzen des Reagens in einem Lösungsmittel erfolgt.

4. Verfahren nach einem der Ansprüche 2 oder 3, dadurch gekennzeichnet, daß die Reaktion zwischen dem 2-Halogen-1,3,4-thiadiazol und dem Amin mit einem Überschuß an Amin durchgeführt wird.

5. Pharmazeutische Zusammensetzung, dadurch gekennzeichnet, daß sie als aktives Ingrediens ein Thiadiazolderivat nach Anspruch 1 in Kombination mit einem pharmazeutisch akzeptablen Vehikel enthält.

6. Pharmazeutische Zusammensetzung, die auf das Zentralnervensystem aktiv ist, dadurch gekennzeichnet, daß sie als aktives Ingrediens ein Thiadiazolderivat nach Anspruch 1 in Kombination mit einem pharmazeutisch akzeptablen Vehikel enthält.

**Patentansprüche für den Vertragsstaat AT:**

1. Verfahren zur Herstellung von Thiadiazolderivaten der allgemeinen Formel I:

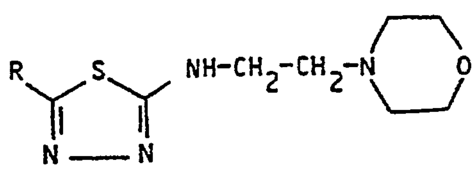

(I)

worin R darstellt:

– eine gerade oder verzweigte Alkylgruppe mit 1 bis 5 Kohlenstoffatomen;
– eine Cycloalkylgruppe mit 5 bis 6 Kohlenstoffatomen;
– eine Phenylgruppe, gegebenenfalls substituiert durch 1 oder 2 Halogenatome, vorzugsweise Chlor, durch 1 oder 2 Alkyl- oder Alkoxygruppen mit 1 bis 5 Kohlenstoffatomen oder durch 1 oder 2 Hydroxygruppen oder durch eine Nitro- oder Trifluormethylgruppe;
– eine Biphenylylgruppe;
– eine alpha-Naphthylgruppe;
und der pharmazeutisch akzeptablen Salze dieser Derivate,
dadurch gekennzeichnet, daß es darin besteht, daß ein 2-Halogen-1,3,4-thiadiazol der Formel

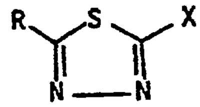

worin X Chlor oder Brom darstellt und R wie oben definiert, aber keine Hydroxyphenyl- oder Dihydroxyphenylgruppe ist, mit einem Amin der Formel

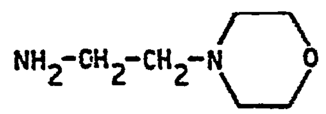

zur Umsetzung gebracht wird, das resultierende Derivat aus dem Reaktionsmilieu extrahiert wird, die erhaltenen Derivate der allgemeinen Formel I, worin R eine Methoxyphenyl- oder Dimethoxyphenylgruppe darstellt, gegebenenfalls nach einem bekannten Verfahren entmethyliert werden, um jene der allgemeinen Formel I zu erhalten, in welcher R für eine Hydroxyphenyl- oder Dihydroxyphenylgruppe steht, und gegebenenfalls die erhaltene Verbindung in eines ihrer pharmazeutisch akzeptablen Salze übergeführt wird.

2. Verfahren nach Anspruch 1, dadurch gekennzeichnet, daß die Reaktion zwischen dem 2-Halogen-1,3,4-thiadiazol und dem Amin durch Erhitzen des Reagens in einem Lösungsmittel erfolgt.

3. Verfahren nach einem der Ansprüche 1 oder 2, dadurch gekennzeichnet, daß die Reaktion zwischen dem 2-Halogen-1,3,4-thiadiazol und dem Amin mit einem Überschuß an Amin durchgeführt wird.

4. Verwendung der durch ein Verfahren nach einem der Ansprüche 1 bis 3 erhaltenen Thiadiazolderivate in Kombination mit einem pharmazeutisch akzeptablen Vehikel zur Herstellung von pharmazeutischen Zusammensetzungen.

5. Verwendung der durch ein Verfahren nach einem der Ansprüche 1 bis 3 erhaltenen Thiadiazolderivate in Kombination mit einem pharmazeutisch akzeptablen Vehikel zur Herstellung von pharmazeutischen Zusammensetzungen, die auf das Zentralnervensystem aktiv sind.